# EUROPEAN PATENT SPECIFICATION

(11) **EP 1 307 586 B1**
(45) Date of publication and mention of the grant of the patent: **07.07.2004**
(21) Application number: 01971812.1
(22) Date of filing: 30.07.2001
(51) Int. Cl.: C12Q 1/26, C07D 311/12

(54) **ENZYME SUBSTRATE**
ENZYMSUBSTRAT
SUBSTRAT D'ENZYME

(30) Priority: 08.08.2000 GB 0019475
(43) Date of publication of application: 07.05.2003
(73) Proprietor: SMITHKLINE BEECHAM PLC, Brentford, Middlesex TW8 9GS (GB)
(72) Inventor: BLOOMER, Jacqueline Carol, Welwyn Hertfordshire AL6 9AR (GB); LEACH, Colin Andrew GlaxoSmithKline, Harlow Essex CM19 5AW (GB)
(74) Representative: Thornley, Rachel Mary
(86) International application number: PCT/EP2001/008788
(87) International publication number: WO 2002/012542

(56) References cited:
- EP-A- 0 897 015
- WO-A-00/08205
- WO-A-00/22159

## Description

This invention relates to compounds, processes for preparing them and their use as enzyme substrates.

The majority of metabolism based drug interactions are a result of inhibition of cytochrome P450 enzymes. Drug interactions involving individual P450 enzymes can be predicted using *in vitro* methods. Typical *in vitro* P450 enzyme assays involve incubation of an appropriate substrate with a source of enzyme. Traditionally, time consuming chromatographic methods have been used for metabolite detection in these incubations. More recently the availability of fluorimetric plate readers has facilitated the higher throughput of enzyme assays in general. Adapting P450 assays to fluorescent plate reader technology requires the identification of substrates with appropriate fluorescent products for individual enzymes. Among the xenobiotic-metabolising cytochromes P450, CYP2C19 and CYP2C9 are two of those responsible for the metabolism of some drugs.

3-Cyano-7-ethoxycoumarin has been described for high throughput CYP2C19 and CYP2C9 inhibition screening (Crespi *et al*, *Anal. Biochem*., 1997, **248**, 188-190). However, the rate of 3-cyano-7-ethoxycoumarin metabolism by CYP2C19 and CYP2C9 is low, therefore more appropriate substrates are required to enable higher throughput inhibition screening.

WO 00/22159 discloses the compounds 7-methoxy-4-trifluoromethyl coumarin-3-acetic acid and 7-ethoxy-4-trifluoromethyl coumarin-3-acetic acid as substrates for CYP2C9.

A compound has now been identified which is an improved substrate for CYP2C19 and CYP2C9 and which is of use for configuring high throughput inhibition screening assays.

According to the invention there is provided an assay for identifying inhibitors of the enzyme CYP2C19 or CYP2C9 which comprises contacting the enzyme and a compound of formula (I): 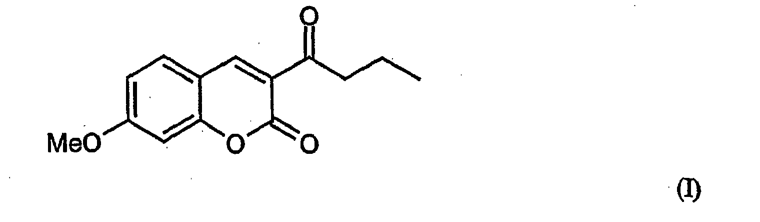 with a test compound and measuring inhibition of O-dealkylation of the compound of formula (I) by the enzyme.

The assay is preferably used for identifying inhibitors of the enzyme CYP2C19.

Generally the rate of O-dealkylation of the compound of formula (I) in the absence of test compound will be known, as will the extent of O-dealkylation at given time points. The assay may test for inhibition of O-dealkylation continuously or at specified time points.

O-Dealkylation of the compound of formula (I) following incubation with CYP2C19 or CYP2C9 gives a readily quantifiable fluorescent product of formula (II): 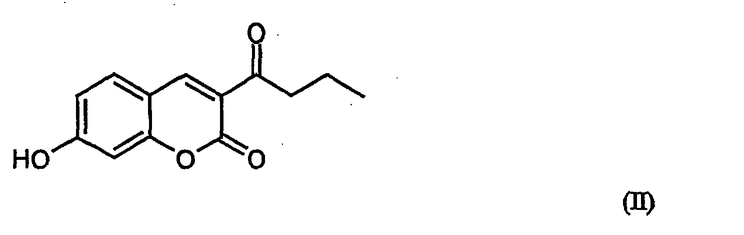 which can be scanned with suitable excitation and emission wavelengths, for example an excitation wavelength of 409 nm and an emission wavelength of 460 nm. Inhibition of O-dealkylation of the compound of formula (I) by the enzyme is preferably measured by quantifying the compound of formula (II).

The assay may be carried out either in solution or utilising a solid support in which case the enzyme may be attached to the solid support. When the assay is carried out in solution suitable solvents include methanol, acetonitrile and DMSO.

The assay is preferably performed in a solution buffered to a pH of 7.4 or 7.5, e.g. using a potassium phosphate or Tris HCl buffer. The assay may also be performed in potassium phosphate buffer containing 10 mM MgCl₂. The assay is preferably performed at a temperature of 37°C.

The test compound may be pre-incubated with enzyme prior to the addition of the substrate, or alternatively the substrate may be added simultaneously with the test compound. Final concentrations of enzyme and substrate are calculated so as to achieve a suitable rate of processing for carrying out the assay. If desired, the reaction may be stopped, for example by addition of acid or solvent.

As will be apparent to those skilled in the art cofactors for the human cytochrome P450 enzyme will be present in the assay system, cofactors for human cytochrome P450 enzymes are NADP, glucose-6-phosphate and glucose-6-dehydrogenase. NADH or NADPH may be used instead of NADP. The assay may conveniently be initiated by addition of the cofactor solution, preferably prewarmed to 37°C, to the test compound / enzyme / substrate mixture.

The fluorescent product of formula (II) may be analysed using any conventional system of fluorescence detection, for example a multi-well plate/fluorescent plate reader.

The compound of formula (I) is novel and as such also forms part of the invention.

The compound of formula (I) may be prepared by conventional methods, for example by methylation of a compound of formula (II) with an alkylating agent such as iodomethane, in the presence of a base such as potassium carbonate. The reaction is preferably performed in a solvent such as dimethylformamide.

Thus according to a further aspect of the invention there is provided a process for the production of a compound of formula (I) which comprises reaction of a compound of formula (II) with a methylating agent, such as iodomethane in the presence of a base such as potassium carbonate.

The compound of formula (II) is commercially available [CAS Registry Number 19491-89-5].

Since the inhibition of cytochrome P450 enzymes is often the mechanism for drug/drug interactions, the assay according to the invention is particularly useful for identifying compounds which may give rise to adverse drug/drug interactions. The assay can therefore be used in combination with the chemical modification of test compounds to increase a test compounds potential for use as a pharmaceutical.

A method for reducing the CYP2C19 or CYP2C9 enzyme inhibitory activity of a compound, comprising the steps of identifying the compound as an inhibitor of CYP2C19 or CYP2C9 in the assay described above; and thereafter producing a chemically modified version of the test compound in which the functionality suspected to be responsible for CYP2C19 or CYP2C9 inhibition is eliminated or changed can produce novel compounds.

The chemical modification of test compounds according to this method can be performed using techniques well known to those skilled in the art.

The novel compounds produced according to this method may find application as pharmaceuticals. A compound produced according to this method will be readily identifiable as novel by performing routine literature and database searches. The pharmaceutical activity of such compounds can be readily ascertained using conventional biological screening methods known to those skilled in the art.

The invention is illustrated by the following examples.

Figure 1 shows the tranylcypromine inhibition of 3-butyryl-7-methoxycoumarin metabolism by CYP2C19.

Figure 2 shows the sulphaphenazole inhibition of 3-butyryl-7-methoxycoumarin metabolism by CYP2C9.

### Example 1

### Preparation of 3-butyryl-7-hydroxycoumarin

A mixture of 2,4-dihydroxybenzaldehyde (4.6 g, 33 mmol) and ethyl butyrylacetate (5.3 ml, 33 mmol) was cooled in an ice bath, and piperidine (1 ml, 10 mmol) was added dropwise with stirring, then the mixture was allowed to warm to room temperature overnight. Acidification with 0.1M hydrochloric acid gave an oily residue, which crystallised from ethanol to give the title compound as a yellow solid (1.8 g, 23%).
δH(d₆-DMSO) 0.91 (3H, t), 1.59 (2H, m), 2.96 (2H, m), 6.76 (1H, m), 6.86 (1H, m), 7.79 (1H, d), 8.59 (1H, s), 11.10 (1H, s);
mass spectrum *m*/*z* 233 (MH⁺).

### Preparation of 3-butyryl-7-methoxycoumarin

Iodomethane (0.224 ml, 3.6 mmol) was added to 3-butyryl-7-hydroxycoumarin (0.70 g, 3 mmol) and potassium carbonate (0.50 g, 3.6 mmol) in dimethylformamide (15 ml), and the mixture was stirred at ambient temperature for 16 hours. A dilute aqueous solution of potassium carbonate was added with vigorous stirring, and the precipitate was filtered off, washed with water, then recrystallised from aqueous ethanol. The title compound was obtained as a white solid (0.54 g, 73%).
δ_{H}(CDCl₃) 0.99 (3H, t), 1.72 (2H, m), 3.09 (2H, m), 3.91 (3H, s), 6.83 (1H, d, J=2Hz), 6.90 (1H, dd, J=2Hz/9Hz), 7.54 (1H, d, J=9H), 8.48 (1H, s);
mass spectrum *m*/*z* 247 (MH⁺).

### Example 2

### Assay methodology for CYP2C19

### Materials:

3.75 mM 3-butyryl-7-methoxycoumarin (i.e. 0.923 mg/mL in DMSO) - store at approx. -20°C in the dark
2 % (w/v) NaHCO₃ - store at approx. 4°C
50 mM potassium phosphate buffer, pH 7.4
Freshly prepared cofactor solution:- approx. the following per mL of 2 % (w/v) NaHCO₃
1.7 mg NADP, monosodium salt
7.8 mg glucose-6-phosphate, monosodium salt
6 Units glucose-6-phosphate dehydrogenase, Type VII from Bakers
Yeast

1) Pre-warm the plate reader oven to 37°C and pre-warm the lamp for at least 10 minutes.
2) Mix 1 µL 3-butyryl-7-methoxycoumarin, 5 µL (50 µg) CYP2C19 microsomal protein and 214 µL buffer per incubate (giving 15 µM 3-butyryl-7-methoxycoumarin and 200 µg/mL protein final concentration).
3) To each well of a 96-well plate add 220 µL of incubation mix and 5 µL of compound (or 5 µL of appropriate solvent for control wells - methanol, acetonitrile or DMSO may be used).
4) Pre-incubate the multi-well plate in the plate reader at 37°C for 5 minutes. Pre-warm the cofactor solution at 37°C for 5 minutes.
5) Add 25 µL cofactor solution to each well and scan with an excitation wavelength of 409 nm and an emission wavelength of 460 nm with a gain of 80. Scan for 10 cycles at 1 minute intervals.

### Results

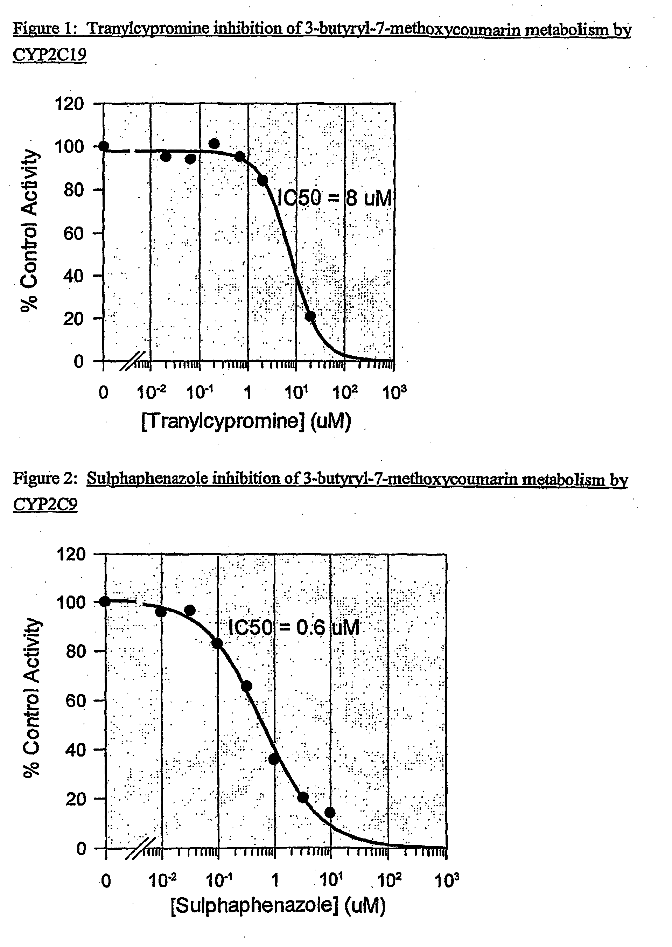

Confirmation of 3-butyryl-7-methoxycoumarin as a CYP2C19 substrate was achieved using tranylcypromine, a diagnostic CYP2C19 inhibitor (Wienkers *et al*, *Drug Metabolism and Disposition*, 1996, **24**(5), 610-614). With tranylcypromine, 3-butyryl-7-methoxycoumarin was inhibited with an IC₅₀ of 8 uM (Figure 1), an inhibition value typical of other, well characterised, CYP2C19 substrates.

### Example 3

### Assay methodology for CYP2C9

### Materials:

5 mM 3-butyryl-7-methoxycoumarin (i.e. 1.23 mg/mL in DMSO) - store at approx. -20°C in the dark
2 % (w/v) NaHCO₃ - store at approx. 4°C
50 mM potassium phosphate buffer, pH 7.4
Freshly prepared cofactor solution:- approx. the following per mL of 2 % (w/v) NaHCO₃
1.7 mg NADP, monosodium salt
7.8 mg glucose-6-phosphate, monosodium salt
6 Units glucose-6-phosphate dehydrogenase, Type VII from Bakers Yeast

1) Pre-warm the plate reader oven to 37°C and pre-warm the lamp for at least 10 minutes.
2) Mix 1 µL 3-butyryl-7-methoxycoumarin, 5 µL (50 µg) CYP2C9 microsomal protein and 214 µL buffer per incubate (giving 20 µM 3-butyryl-7-methoxycoumarin and 200 µg/mL protein final concentration).
3) To each well of a 96-well plate add 220 µL of incubation mix and 5 µL of compound (or 5 µL of appropriate solvent for control wells - methanol, acetonitrile or DMSO may be used).
4) Pre-incubate the multi-well plate in the plate reader at 37°C for 5 minutes. Pre-warm the cofactor solution at 37°C for 5 minutes.
5) Add 25 µL cofactor solution to each well and scan with an excitation wavelength of 409 nm and an emission wavelength of 460 nm with a gain of 80. Scan for 10 cycles at 1 minute intervals.

### Results

Confirmation of 3-butyryl-7-methoxycoumarin as a CYP2C9 substrate was achieved using sulphaphenazole, a diagnostic CYP2C9 inhibitor (Back *et al*, *British Journal of Clinical Pharmacology*, 1988, **26**, 23-29). With sulphaphenazole, 3-butyryl-7-methoxycoumarin was inhibited with an IC₅₀ of 0.6 uM (Figure 2), an inhibition value typical of other, well characterised, CYP2C9 substrates.

## Claims

1. An assay for identifying inhibitors of the enzyme CYP2C19 or CYP2C9 which comprises contacting the enzyme and a compound of formula (I): 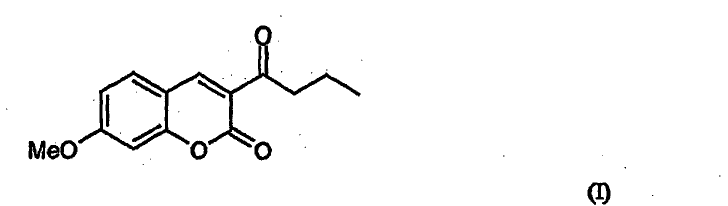 with a test compound and measuring inhibition of O-dealkylation of the compound of formula (I) by the enzyme.

2. The assay according to claim 1 for identifying inhibitors of the enzyme CYP2C19.

3. The assay according to claim 1 or 2 wherein inhibition of O-dealkylation of the compound of formula (I) by the enzyme is measured by quantifying the compound of formula (II):

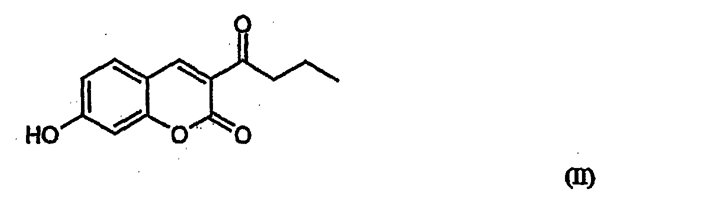

4. The assay according to claim 3 wherein the compound of formula (II) is quantified by fluorescence detection.

5. The assay according to claim 4 wherein the compound of formula (II) is quantified by scanning at excitation wavelength of 409 nm and an emission wavelength of 460 nm.

6. A compound of formula (I) as defined in claim 1.

7. A process for the production of a compound of formula (I) as defined in claim 1 which comprises reaction of a compound of formula (II) with a methylating agent.

## Patentansprüche

1. Assay zur Identifizierung von Inhibitoren des Enzyms CYP2C19 oder CYP2C9, welches das in Kontakt bringen des Enzyms und einer Verbindung der Formel (I): 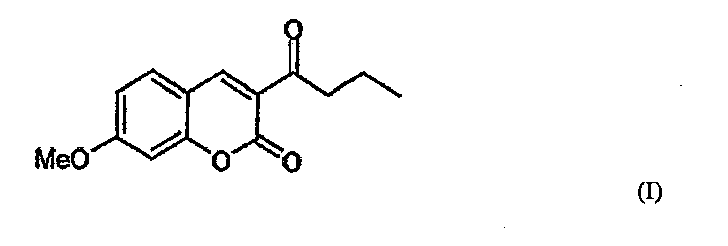 mit einer Testverbindung und die Messung der Inhibierung der O-Dealkylierung der Verbindung der Formel (I) durch das Enzym umfasst.

2. Assay gemäß Anspruch 1 zur Identifizierung von Inhibitoren des Enzyms CYP2C19.

3. Assay gemäß Anspruch 1 oder 2, wobei die Inhibierung der O-Dealkylierung der Verbindung der Formel (I) durch das Enzym durch die quantitative Bestimmung der Verbindung der Formel (II) gemessen wird:

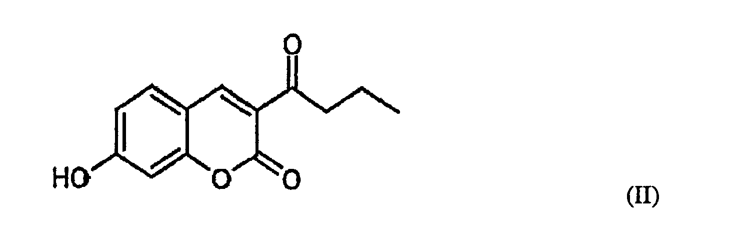

4. Assay gemäß Anspruch 3, wobei die Verbindung der Formel (II) quantitativ mit Fluoreszenzdetektion bestimmt wird.

5. Assay gemäß Anspruch 4, wobei die Verbindung der Formel (II) quantitativ durch Scannen bei einer Anregungswellenlänge von 409 nm und einer Emissionswellenlänge von 460 nm bestimmt wird.

6. Verbindung der Formel (I), die die in Anspruch 1 angegebene Bedeutung hat.

7. Verfahren zur Herstellung einer Verbindung der Formel (I), die die in Anspruch 1 angegebene Bedeutung hat, das das Umsetzen einer Verbindung der Formel (II) mit einem Methylierungsmittel umfasst.

## Revendications

1. Analyse pour l'identification d'inhibiteurs de l'enzyme CYP2C19 ou CYP2C9, qui comprend la mise en contact de l'enzyme et d'un composé de formule (I) : 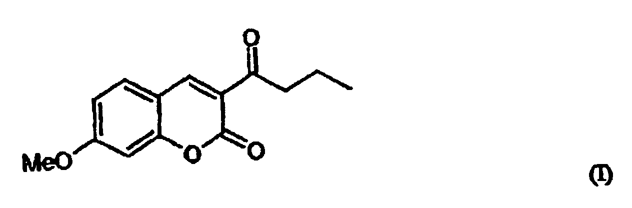 avec un composé d'essai et la mesure de l'inhibition de la O-désalkylation du composé de formule (I) par l'enzyme.

2. Analyse suivant la revendication 1, pour l'identification d'inhibiteurs de l'enzyme CYP2C19.

3. Analyse suivant la revendication 1 ou 2, dans laquelle l'inhibition de la O-désalkylation du composé de formule (I) par l'enzyme est mesurée en quantifiant le composé de formule (II) :

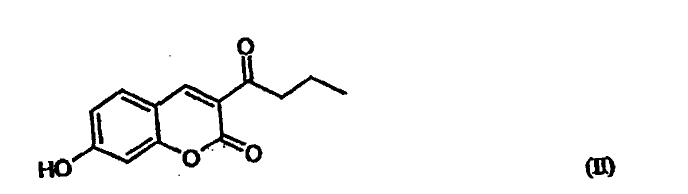

4. Analyse suivant la revendication 3, dans laquelle le composé de formule (II) est quantifié par détection de fluorescence.

5. Analyse suivant la revendication 4, dans laquelle le composé de formule (II) est quantifié par balayage à une longueur d'onde d'excitation de 409 nm et une longueur d'onde d'émission de 460 nm.

6. Composé de formule (I) répondant à la définition suivant la revendication 1.

7. Procédé pour la production d'un composé de formule (I) répondant à la définition suivant la revendication 1, qui comprend la réaction d'un composé de formule (II) avec un agent de méthylation.
